# EUROPEAN PATENT SPECIFICATION

(11) **EP 2 413 777 B1**
(45) Date of publication and mention of the grant of the patent: **31.12.2014**
(21) Application number: 10713724.2
(22) Date of filing: 29.03.2010
(51) Int. Cl.: A61B 1/005, A61B 1/31, A61B 1/00, A61B 5/06, A61B 6/12, A61B 6/00, A61B 6/03, A61B 5/055, A61B 19/00

(54) **ASSOCIATING A SENSOR POSITION WITH AN IMAGE POSITION**
ASSOZIATION EINER SENSORPOSITION MIT EINER BILDPOSITION
ASSOCIATION D'UNE POSITION DE CAPTEUR À UNE POSITION D'IMAGE

(30) Priority: 03.04.2009 EP 09157290
(43) Date of publication of application: 08.02.2012
(73) Proprietor: Koninklijke Philips N.V., 5656 AE Eindhoven (NL)
(72) Inventor: TRUYEN, Roel, NL-5656 AE Eindhoven (NL)
(74) Representative: van Velzen, Maaike Mathilde
(86) International application number: PCT/IB2010/051347
(87) International publication number: WO 2010/113097

(56) References cited:
- WO-A2-2007/025081
- US-A1- 2005 182 319
- US-A1- 2007 078 334
- VRIES DE A H ET AL: "FEASIBILITY OF AUTOMATED MATCHING OF SUPINE AND PRONE CT-COLONOGRAPHY EXAMINATIONS" BRITISH JOURNAL OF RADIOLOGY, BRITISH INSTITUTE OF RADIOLOGY, LONDON, GB LNKD- DOI:10.1259/BJR/55953054, vol. 79, no. 945, 1 January 2006 (2006-01-01), pages 740-744, XP008068756 ISSN: 0007-1285 cited in the application

## Description

### FIELD OF THE INVENTION

The invention relates to associating a sensor position with an image position. The invention also relates to associating a colonoscope tip position with a position in a medical image.

### BACKGROUND OF THE INVENTION

US 2007/0078334 discloses a DC magnetic-based position and orientation monitoring system for tracking medical instruments. It follows 3D sensor tip locations superimposed on anatomical images reconstructed into 3D volumetric computer models. Sensor data can be integrated with real-time imaging modalities, such as endoscopes, for intrabody navigation of instruments with instantaneous feedback through critical anatomy to locate and remove tissue. Registration is based on touching multiple points in image space and patient space, these points being anatomical landmarks (skeletal structures) or fiducial markers affixed to the patient. The registration algorithm accounts for shifts, rotations, and scaling of points from one frame to another.

US 2005/0182319 A1 discloses an anatomical region of interest being imaged using an imaging device such as an x-ray device. A tracked registration device may then be removably inserted in a conduit within the anatomical region and the position of the registration device may be sampled by a tracking device as the registration device is moved within the anatomical region through the catheter. The sampled position data is registered to the image data to register the path of the conduit to the anatomical region of interest.

However, the association between the sensor and the image positions is not sufficiently reliable.

Document WO2007/025081 discloses a system and a method according to the preamble of claims 1 and 10, respectively.

### SUMMARY OF THE INVENTION

The invention is set out in claims 1 and 10.

It would be advantageous to have an improved system for associating a sensor position with an image position. To better address this concern, in a first aspect of the invention a system is presented that comprises
- position information means for obtaining position information relating to a sequence of sensor positions inside a tubular structure, the position information being indicative of a sensor position relative to a structural characteristic of the tubular structure; and
- matching means for matching the sequence of sensor positions with a corresponding sequence of image positions, the image positions being indicative of positions inside a corresponding tubular structure represented by an image, based on the position information and using the structural characteristic of the tubular structure, for obtaining matching information.

The shape of the tubular structure may deform between the time of acquiring the image and the time of obtaining the position information. However, such deformation is not taken into account by the known registration algorithm, which uses the position information based on fiducial markers affixed to the patient and/or anatomical landmarks based on skeletal structures. The proposed system provides position information indicative of a sensor position relative to a structural characteristic of the tubular structure itself. Consequently, the matching means matches the sensor positions with the corresponding sequence of image positions, using this sensor position relative to the structural characteristic of the tubular structure. Consequently, the matching is less sensitive to any deformations of the tubular structure. Even if such a deformation has occurred, the sensor position can be matched with the image position, based on the information relative to the structural characteristic of the tubular structure. Preferably, the structural characteristic can be identified in both the tubular structure at the time the sensor is inside and the corresponding tubular structure represented by the image.

The system may comprise associating means for associating a data element measured by the sensor at a particular sensor position inside the tubular structure with a corresponding image position in the corresponding tubular structure, based on the matching information. By using the matching information, a particular sensor position can be associated with the corresponding image position relatively easily, taking into account a deformation of the tubular structure. By using the matching information, the image position corresponding to the position where the data was acquired can be identified and associated with the sensor data. The data element may comprise an image, for example in the case the sensor comprises an optical endoscope, the data element may comprise an optical image acquired at a particular position inside the tubular structure.

The system may comprise visualizing means for visualizing an indication of the data element and an indication of the corresponding image position. Visualizing said two indications allows a user to identify the image position to which the data element belongs.

The visualizing means may be responsive to position information relating to a current position of the sensor. This allows the user to identify the image position corresponding to the current position of the sensor. Such information is helpful during an intervention, for example for navigating to a particular lesion identified in the image.

The system may comprise reporting means for creating a report comprising the indication of the data element and the indication of the corresponding image position. Such reporting is facilitated by the matching information.

The tubular structure comprises a colon. In such a case the sensor position inside the colon may be associated with an image position. The colon is known to change shape from time to time. The matching information helps to find the corresponding image position even when the colon has changed shape between the time of acquiring the image and the time of endoscopy.

The sensor comprises an endoscope, for example a colonoscope. Such an endoscope can acquire data elements in the form of images inside a tubular structure. The endoscope may comprise an optical endoscope for obtaining optical images. Alternatively, other kinds of endoscopes may be used, using for example infrared imaging.

The position information is indicative of a sensor position relative to a segment boundary between two segments of the colon. Segment boundaries may include boundaries between such colon segments as the caecum, ascending colon, transverse colon, descending colon, sigmoid, rectum. Such segment boundaries can be determined relatively easily by means of a sensor, in particular an endoscope.

The position information comprises an indication of when the sensor crosses a segment boundary. By indicating when the sensor crosses a segment boundary, the position of the segment boundary is indicated relatively accurately. Moreover, such indication is relatively easy to provide manually or in automated fashion. Moreover, an indication of corresponding segment boundaries in the image can be made, which helps the matching process.

Position information is obtained at least in part by interpolating between two segment boundaries, based on a traversing speed of the sensor. Information of the traversing speed of the sensor may be made available, for example information indicating that the traversing speed is fixed, or is variable but known. Such information can be used to interpolate the positions between two segment boundaries.

The position information may comprise spatial coordinates of the sensor. Such spatial coordinates provide relatively accurate information of the position of the sensor. Moreover, such spatial coordinates may be established for any position of the sensor during an intervention, independent of any traversing speed. Such spatial coordinates are for example helpful for obtaining position information during manual guidance of the endoscope.

A sequence of spatial coordinates may be associated with the sequence of sensor positions. This sequence of spatial coordinates may be indicative of at least part of a centerline of the tubular structure. The structural characteristic may comprise a shape of the at least part of the centerline. Such a sequence of spatial coordinates provides relatively detailed information of the structural characteristic. This may enhance the accuracy.

The matching means may comprise aligning means for aligning at least part of the centerline of the tubular structure with at least part of a centerline of the corresponding tubular structure represented by the image. This way relatively accurate matching information may be obtained.

The aligning means may comprise means for locally stretching or compressing a centerline for improving a similarity between at least part of the centerline of the tubular structure and at least part of the centerline of the corresponding tubular structure represented by the image. Such stretching or compressing of a centerline is a suitable operation when aligning two centerlines of a colon, wherein the colon may have deformed.

An image acquisition apparatus may comprise an embodiment of the system according to the invention.

A method of associating a sensor position with an image position comprises
- obtaining position information relating to a sequence of sensor positions inside a tubular structure, the position information being indicative of a sensor position relative to a structural characteristic of the tubular structure; and
- matching the sequence of sensor positions with a corresponding sequence of image positions, the image positions being indicative of positions inside a corresponding tubular structure represented by an image, based on the position information and using the structural characteristic of the tubular structure, for obtaining matching information.

A computer program product may comprise instructions for causing a processor system to perform the steps of the method set forth.

It will be appreciated by those skilled in the art that two or more of the above-mentioned embodiments, implementations, and/or aspects of the invention may be combined in any way deemed useful.

Modifications and variations of the image acquisition apparatus, of the workstation, of the system, and/or of the computer program product, which correspond to the described modifications and variations of the system, can be carried out by a person skilled in the art on the basis of the present description.

A person skilled in the art will appreciate that the method may be applied to multidimensional image data, e.g., to 2-dimensional (2-D), 3-dimensional (3-D) or 4-dimensional (4-D) images, acquired by various acquisition modalities such as, but not limited to, standard X-ray Imaging, Computed Tomography (CT), Magnetic Resonance Imaging (MRI), Ultrasound (US), Positron Emission Tomography (PET), Single Photon Emission Computed Tomography (SPECT), and Nuclear Medicine (NM).

### BRIEF DESCRIPTION OF THE DRAWINGS

These and other aspects of the invention will be further elucidated and described with reference to the drawing, in which
Fig. 1 is a block diagram illustrating a system for associating a sensor position with an image position.
Fig. 2 is a diagram of a display showing an association between a sensor position and an image position;
Fig. 3 is a drawing of a colon;
Fig. 4 is a block diagram illustrating a method of associating a sensor position with an image position.

### DETAILED DESCRIPTION OF EMBODIMENTS

The Figures and embodiments are described herein for the purpose of illustration. They do not limit the scope of the invention.

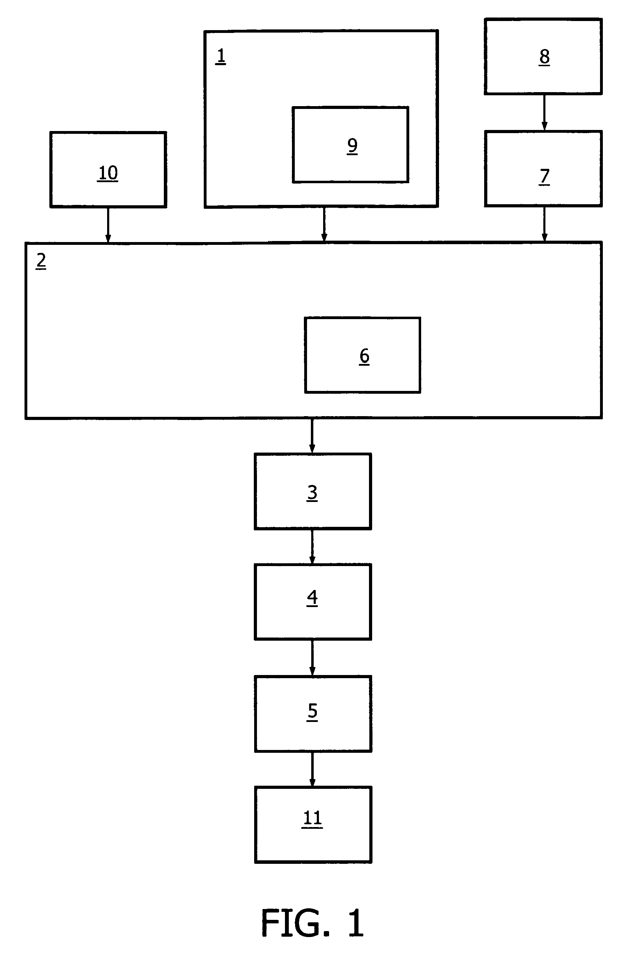

Fig. 1 illustrates a system for associating a sensor position with an image position. The system may be implemented at least in part using a workstation. Such a workstation may comprise a processing unit, a working memory (RAM memory), and a permanent storage means such as a flash memory, or a magnetic hard disk. Input and output means are also provided, for example a network connection for receiving image data and sensor data as well as position information. The network connection may be used to transmit data such as data associating sensor data with an image position or a report comprising sensor data and an image in which the corresponding image position is indicated. The data may also be provided by means of a removable storage medium such as CD-ROM, for example. A display may be provided for displaying for example sensor data and image data and their associations. An input, such as a keyboard, mouse, trackball, microphone, may be provided for controlling the operation of the system. The microphone may be used, for example, to receive voice commands. To interpret the voice commands, voice command processing software may be provided. Voice commands may be used, for example, to indicate a segment boundary or to visualize a position in an image, corresponding to the sensor position. The image data may comprise, for example, computed tomography (CT) data or magnetic resonance imaging (MRI) data. The image may also comprise an x-ray image.

The system may comprise a sensor 10 suitable for obtaining sensor data from inside a tubular structure. Such a sensor comprises an endoscope. The sensor may be connected to a wire which may be used to push or pull the endoscope further into or out of the tubular structure.

The system comprises position information means 1 for obtaining position information relating to a sequence of sensor positions inside a tubular structure, the position information being indicative of a sensor position relative to a structural characteristic of the tubular structure. The structural characteristic of the tubular structure may comprise an aspect of the shape of the tubular structure. For example, a characteristic bend of a tubular structure. Such a characteristic may be based on the centerline of the tubular structure or a path inside the tubular structure. The tubular structure comprises a shape characteristic, which can be observed by means of the sensor 10 and is a colon segment boundary.

The tubular structure comprises the colon 30.

The system may comprise an image acquisition apparatus 8 for providing the image data such as CT data or MRI data. Alternatively, the image data is retrieved from an external source such as an external image acquisition apparatus, or a data server on the network, for example using a PACS.

The system may comprise image position information means 7 for identifying a sequence of image positions in the image. For example, image position information means 7 comprises a user interface which enables a user to indicate the image positions interactively, using a pointing device such as a mouse or trackball. Alternatively, image processing software may be provided to automatically identify the image positions. The image positions may be points, of which the relative position to a structural characteristic of the tubular structure is known. For example, the points may comprise segment boundaries of a colon. Alternatively, the positions define a centerline of the tubular structure; the structural characteristic may comprise particular bends known to exist in the tubular structure.

The system comprises matching means 2 for matching the sequence of sensor positions with a corresponding sequence of image positions. This corresponding sequence of image positions may be obtained from image position information means 7. The image positions may be indicative of positions inside a corresponding tubular structure represented by the image. The matching means 2 performs the matching based on the position information. In the matching process, the structural characteristic of the tubular structure is used for obtaining matching information.

The system may comprise associating means 3 for associating a data element measured by the sensor 10 at a particular sensor position inside the tubular structure with a corresponding image position in the corresponding tubular structure, based on the matching information. Said association may be used for visualizing or reporting means, for example. The association may also be used for automatic navigation systems.

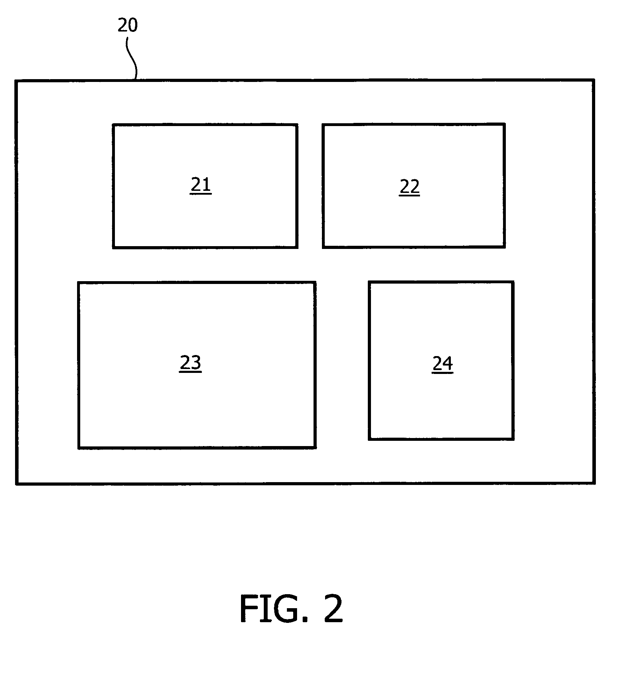

The system may further comprise visualizing means 4 for visualizing an indication of the data element and an indication of the corresponding image position. Such indication reveals the association to a user. An example is given in Fig. 2.

Fig. 2 illustrates a display 20 comprising sensor data, for example an optical colonoscopy image 21 acquired at a particular sensor position. At the same time, the display 20 shows a virtual endoscopy image 22. The virtual endoscopy image 22 is a view generated from the image data. The virtual endoscopy image 22 represents a reconstruction of the image data 'as seen' from the image position corresponding to the particular sensor position. The display 20 further shows an overview 23 of at least part of the tubular structure as represented by the image. The image position corresponding to the particular sensor position is indicated, by means of highlighting or by means of a symbol such as an arrow, for example. The display 20 further comprises a list 24 of points of interest, for example a list of lesions or a list of image findings. The nearest point of interest may be indicated in the list 24. Moreover, the image positions of these points of interest may also be indicated in the overview 23 and/or the virtual endoscopy image 22.

Returning to Fig. 1, the visualizing means 4 may be responsive to position information relating to a current position of the sensor 10. For example, the sensor position may be provided by the position information means 1 in real-time. The visualizing means 4 may be arranged to display and/or update one or more of the views illustrated with respect to Fig. 2 in real-time.

The system may comprise reporting means 5 for creating a report comprising the indication of the data element and the indication of the corresponding image position. The report may comprise one or more, or all, of the elements described in relation to Fig. 2. The report may also comprise annotations attached to particular sensor positions, sensor data, and/or image positions. The reporting means may comprise user interfaces for enabling a user to input such annotations. The reporting means may comprise means for exporting the report for example by the network or for printing the report.

The position information means 1 may be arranged for enabling a user to indicate a segment boundary relative to the sensor, for example to indicate when the sensor 10 crosses such a segment boundary. The position information means 1 may also be arranged for providing such indication automatically, using signal processing techniques. Such a segment boundary may be visible, for example in an acquired image, if optical colonoscopy is used. The position information means 1 is further be arranged to establish, by means of time interpolation, the relative position of the sensor between two successive segment boundaries. This is based on a traversing speed of the sensor. To provide the interpolation, the time of crossing a first segment boundary, the time when the sensor reaches a particular position, and the time of crossing a second segment boundary are obtained. These times allow establishing a relative position within a colon segment. The matching means matches the points corresponding with the segment boundaries for obtaining the matching information. Next, the relative position within a colon segment is used to find the relative position within the colon segment in the image. The result is a corresponding image position.

The position information may comprise spatial coordinates of the sensor 10. For example, the position information means 1 comprises an object localization system 9. The object localization system may comprise an electromagnetic tracking system. The sensor may comprise an electromagnetic marker, of which the electromagnetic tracking system can provide spatial coordinates. This marker is for example attached to a colonoscope tip, so that spatial coordinates of the colonoscope tip may be obtained.

The position information means 1 may be arranged for providing a sequence of spatial coordinates associated with a sequence of sensor positions. Such a sequence of spatial coordinates may be indicative of at least part of a centerline of the tubular structure. For example, the sensor is tracked while it traverses the tubular structure. The structural characteristic used by the matching means 2 may comprise a shape of the at least part of the centerline.

The matching means 2 may comprise aligning means 6 for aligning at least part of the centerline of the tubular structure with at least part of a centerline of the corresponding tubular structure represented by the image. The aligning means may use registration techniques. For example, a matching technique which is known for matching the centerlines of the colon as acquired in a prone CT scan and a supine CT scan may be used to align the centerline of the tubular structure established by tracking the sensor position with at least part of the centerline of the corresponding tubular structure represented by the image. This is explained in more detail elsewhere in this description. For example, the aligning means 6 may comprise means for locally stretching or compressing a centerline for improving a similarity between at least part of the centerline of the tubular structure and at least part of the centerline of the corresponding tubular structure represented by the image.

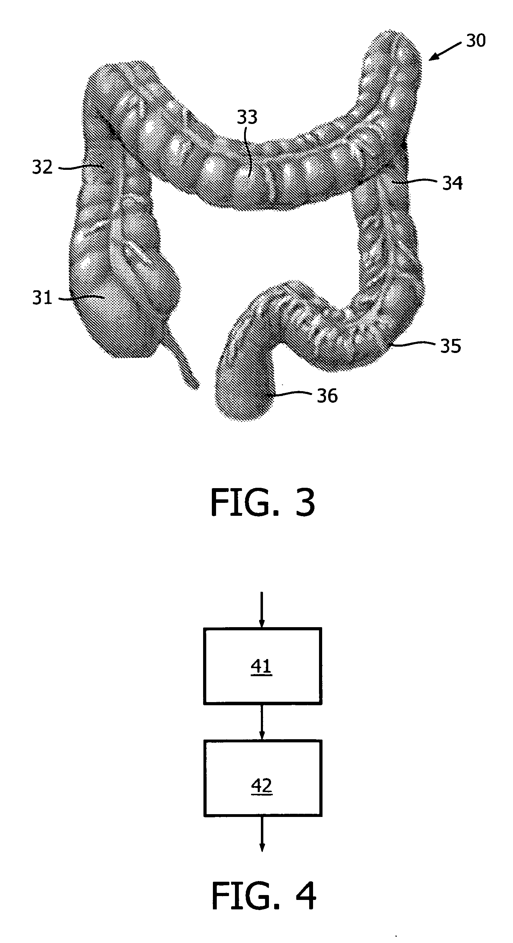

Fig. 4 illustrates a method of associating a sensor position with an image position. The method comprises a step 41 of obtaining position information relating to a sequence of sensor positions inside a tubular structure, the position information being indicative of a sensor position relative to a structural characteristic of the tubular structure. Moreover, the method comprises a step 42 of matching the sequence of sensor positions with a corresponding sequence of image positions, the image positions being indicative of positions inside a corresponding tubular structure represented by an image, based on the position information and using the structural characteristic of the tubular structure, for obtaining matching information. A computer program product may comprise instructions for causing a processor system to perform the steps of said method.

Findings identified in volumetric images (e.g. CT or MR colonoscopy) may be used as an aid in performing optical endoscopy. Also, volumetric images and optical endoscopy images may be combined to perform multimodality reporting. Such applications may benefit from improved correspondence between the volumetric image and the optical endoscopy images. In the following, an example of CT colonography will be described in detail. In this example, polyps may be detected and reported/annotated on the CT images by a radiologist. After that, the gastroenterologist may perform an optical colonoscopy to remove or biopsy these polyps. However, this example is not to be construed as limiting the invention. It should be clear that other volumetric images (like MR) can be used. Moreover, the invention may be applied to other applications, such as combining volumetric and endoscopic data collection.

Polyps may be detected in a patient, using optical colonoscopy (OC). OC may also be used to remove or biopsy the polyps. CT colonography (also known as virtual colonoscopy) is another technique to detect polyps in the colon. Colon cancer is often preceded by the presence of a polyp before it becomes malignant. In order to detect polyps in an early stage, a minimally invasive CT scan may be taken, which allows the radiologist to detect clinically significant polyps.

Navigation in optical endoscopy may be performed based on the optical images taken by the endoscope itself, and using the expert's anatomical knowledge to determine where the endoscope tip is located. In practice, it is not always clear in what colon segment the endoscope is located. The location of the endoscope may be important when the clinician has information on the location of the polyps from a different modality, such as CT or MRI scans. A problem arises when a polyp identified on CT may be missed on optical endoscopy because it is not clear for the gastroenterologist where exactly it is located. Just the segment information may not be sufficient in that case. Also, finding back a known CT polyp in optical colonoscopy may be very time consuming, because of location uncertainty.

If the CT and optical colonoscopy results are not reported together, it may be difficult or impossible to relate the biopsy information (such as the pathology of the removed polyp) with the coordinates of the CT image. Successive scans of the patient may be made after the colonoscopy. Such scans may be used for staging or follow-up. However, it may be difficult or impossible to find the location of the removed polyp in such successive scans.

An application of registering an endoscope tip with an image location is described hereinafter. A display may be provided on which live optical colonoscopy images may be shown. Moreover, a list of lesions (polyps or tumors) that were found in CT may be shown. An overview image may show the colon and indicate the lesions. An endoluminal view of a polyp may be shown based on the CT data. Such a view may mimic the view of the inside of the colon as seen through the optical endoscope (top right). A multiplanar reformat view showing grey values of the CT image may be useful to show which parts of the image are stool.

One polyp may be selected and indicated as such. Several properties of this lesion may be shown: size and location in the lesion list, position in the overview image, size and morphology in the endoluminal view, and structure and grey values in the multiplanar reformat. This view may show any other information relevant for the gastroenterologist.

Selection of a polyp (e.g. if multiple polyps were found in the CT dataset) can be done by the gastroenterologist using e.g. voice commands or by using user interface elements provided on the endoscope.

The location of the endoscope tip can be combined with the CT data in several ways. For example, it is possible to indicate the position of the endoscope tip, using a graphics symbol in the CT overview image to show where the endoscope is located in the CT overview of the colon. The same holds for other CT images such as an endoluminal view or multiplanar reformat. It is also possible to interactively update the views generated from the CT images, based on the endoscope tip position. For example, if the endoscope tip moves, the endoluminal view of the CT image may be redrawn using a camera position corresponding to the position of the endoscope tip.

Reporting may be done using an annotation user interface. Such a user interface may show on a display any of the views described above. For example, images or video images recorded by the endoscope may be shown, as well as an overview image of the CT data, an endoluminal view of the CT data, or a multiplanar reformat view of the CT data. The views may be linked such that they relate to the same location in the colon. Additional diagnostic information observed from the colonoscopy (such as morphology, polypectomy specimen number) can be added to the report, where it is appropriately linked to a particular position in the CT data and/or linked to one or more images of the colonoscopy. When a polyp has been found on optical colonoscopy, it can be linked to a polyp identified in CT. Consequently, it is possible to provide a report in which a lesion is identified in both a CT image and in a colonoscopy image. Moreover, pathology findings and follow-up information can easily be added to the report later on.

Fig. 3 illustrates a colon 30 which comprises 6 segments. These segments are the caecum 31, the ascending colon 32, the transverse colon 33, the descending colon 34, the sigmoid 35, and the rectum 36. The boundary between the ascending colon and the transverse colon and the boundary between the transverse colon and the descending colon are called the flexura. These boundaries can be indicated by the radiologist when reading the CT scan. They can also be identified automatically using image processing.

During colonoscopy, the gastroenterologist can indicate when the colonoscope crosses a segment boundary. The caecum 31 (start of the colon) and the two flexura are relatively easy to identify during optical colonoscopy. Also the position of the rectum is relatively easy to indicate.

Corresponding annotated segment boundaries can be mapped between a medical image and colonoscopy, and from that the correspondences between the other points of the colon in the medical image and in colonoscopy can be calculated. For example, a linear interpolation between the known corresponding points can be performed.

It is also possible to track 3D coordinates of the endoscope. This can be done using electromagnetic (EM) tracking. Electromagnetic tracking is known in the art per se. For example, the PercuNav system marketed by Traxtal Inc. (Toronto ON, Canada) may be used. To this end, an electromagnetic marker may be attached to or formed by the tip of the endoscope. However, other techniques to track the 3D coordinates of the endoscope may be used instead. For example, image processing techniques may be used. By imaging the endoscope tip from multiple directions (using x-ray fluoroscopy, for example), the endoscope tip may be localized in a 3D coordinate system. Such image based localization techniques are known in the art per se.

This way, the 3D coordinates of the tip of the endoscope can be tracked. These 3D coordinates may be recorded in a coordinate reference system attached to the EM tracking device that is not related to the patient coordinate system used for the CT scans. However, a method is provided to register the 3D EM tracker coordinates with the coordinates of the colon centerline in the 3D scan. Such registration may be performed without the need for calibrating the system. In particular, it may be unnecessary to manually define landmarks in patient space.

In the article "Feasibility of automated matching of supine and prone CT-colonography examinations", by A.H. de Vries, R. Truyen, J. van der Peijl, J. Florie, R.E. van Gelder, F.A. Gerritsen, and J Stoker, which appeared in the British Journal of Radiology (2006) 79, pp.740-744, a matching technique was disclosed for matching the centerlines of the colon as acquired in a prone CT scan and a supine CT scan, respectively. In prone-supine matching, the centerlines of two such scans can be aligned by locally stretching or compressing the centerlines so that they are as similar as possible. Similarity can be measured using the difference in local 3D direction of both centerlines. This technique can cope with local deformations of the centerline and can perform a mapping between both centerlines even if the colon has deformed substantially between the two scans. Such deformations can be due to activity of the muscles of the colon. The matching technique described in the paper by De Vries et al. can be applied to match a centerline obtained from colonoscopy, as will be described hereinafter.

It is possible to define two paths which can be mapped using a matching technique as described in De Vries et al., for example. However, other matching techniques may also be used. One of the paths to be matched is based on a plurality of 3D coordinates of the endoscope tip. These 3D coordinates may be established while inserting the colonoscope, or during pull-back of the colonoscope. The other path is based on image data (for example a prone or a supine CT scan). These two paths may be matched to each other. The tracked endoscope coordinates may be more or less restricted to the colon lumen, and may thus have similar shape properties as a colon centerline. Since the colon may deform in a particular way, a matching algorithm for matching colon centerlines may be used to register an endoscope tip trajectory with a colon centerline obtained from a medical image.

It will be appreciated that the invention also extends to computer programs, particularly computer programs on or in a carrier, adapted for putting the invention into practice. The program may be in the form of a source code, an object code, a code intermediate source and object code such as a partially compiled form, or in any other form suitable for use in the implementation of the method according to the invention. It will also be appreciated that such a program may have many different architectural designs. For example, a program code implementing the functionality of the method or system according to the invention may be subdivided into one or more subroutines. Many different ways to distribute the functionality among these subroutines will be apparent to the skilled person. The subroutines may be stored together in one executable file to form a self-contained program. Such an executable file may comprise computer executable instructions, for example processor instructions and/or interpreter instructions (e.g. Java interpreter instructions). Alternatively, one or more or all of the subroutines may be stored in at least one external library file and linked with a main program either statically or dynamically, e.g. at run-time. The main program contains at least one call to at least one of the subroutines. Also, the subroutines may comprise function calls to each other. An embodiment relating to a computer program product comprises computer executable instructions corresponding to each of the processing steps of at least one of the methods set forth. These instructions may be subdivided into subroutines and/or stored in one or more files that may be linked statically or dynamically. Another embodiment relating to a computer program product comprises computer executable instructions corresponding to each of the means of at least one of the systems and/or products set forth. These instructions may be subdivided into subroutines and/or stored in one or more files that may be linked statically or dynamically.

The carrier of a computer program may be any entity or device capable of carrying the program. For example, the carrier may include a storage medium, such as a ROM, for example a CD ROM or a semiconductor ROM, or a magnetic recording medium, for example a floppy disc or hard disk. Further the carrier may be a transmissible carrier such as an electrical or optical signal, which may be conveyed via electrical or optical cable or by radio or other means. When the program is embodied in such a signal, the carrier may be constituted by such a cable or other device or means. Alternatively, the carrier may be an integrated circuit in which the program is embedded, the integrated circuit being adapted for performing, or for use in the performance of, the relevant method.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system for associating a sensor positipn with an image position, comprising:
- position information means (1) arranged for obtaining position information of a sequence of sensor positions of a sensor (10) inside a tubular structure, the tubular structure comprising a colon, the sensor (10) comprising an endoscope, a sensor position being relative to a structural characteristic of the tubular structure; and
- matching means (2) arranged for, based on the position information and using the structural characteristic of the tubular structure, matching the sequence of sensor positions with a corresponding sequence of image positions, the image positions being positions inside a corresponding tubular structure represented by an image and the image positions being known relative to the structural characteristic of the tubular structure, for obtaining matching information;
**characterized by**:
- the structural characteristic of the tubular structure being a segment boundary between two segments of the colon, the position information comprising an indication of when the sensor (10) crosses the segment boundary, and
- the position information means (1) being arranged for establishing, by means of time interpolation, a relative position of the sensor (10) between two segment boundaries, based on a traversing speed of the sensor (10) and the position information.

2. The system according to claim 1, further comprising associating means (3) arranged for associating a data element (21) measured by the sensor (10) at a particular sensor position inside the tubular structure with a corresponding image position (22) in the corresponding tubular structure, based on the matching information.

3. The system according to claim 2, further comprising visualizing means (4) arranged for visualizing an indication of the data element (21) and an indication of the corresponding image position (22).

4. The system according to claim 3, the visualizing means (4) being responsive to position information relating to a current position of the sensor (10).

5. The system according to claim 3, further comprising reporting means (5) arranged for creating a report comprising the indication of the data element (21) and the indication of the corresponding image position (22).

6. The system according to claim 1, the position information (1) comprising spatial coordinates of the sensor (10), a sequence of spatial coordinates associated with the sequence of sensor positions being indicative of at least part of a centerline of the tubular structure, the structural characteristic comprising a shape of the at least part of the centerline.

7. The system according to claim 6, the matching means (2) comprising aligning means (6) arranged for aligning at least part of the centerline of the tubular structure with at least part of a centerline of the corresponding tubular structure represented by the image.

8. The system according to claim 7, the aligning means (6) comprising means arranged for locally stretching or compressing a centerline for improving a similarity between at least part of the centerline of the tubular structure and at least part of the centerline of the corresponding tubular structure represented by the image.

9. An image acquisition apparatus comprising the system according to any one of the preceding claims.

10. A method of associating a sensor position with an image position, comprising:
- obtaining (41) position information of a sequence of sensor positions of a sensor (10) inside a tubular structure, the tubular structure comprising a colon, the sensor comprising an endoscope, a sensor position being relative to a structural characteristic of the tubular structure; and
- based on the position information and using the structural characteristic of the tubular structure, matching (42) the sequence of sensor positions with a corresponding sequence of image positions, the image positions being positions inside a corresponding tubular structure represented by an image and the image positions being known relative to the structural characteristic of the tubular structure, for obtaining matching information;
**characterized by**: the structural characteristic of the tubular structure being a segment boundary between two segments of the colon, the position information comprising an indication of when the sensor (10) crosses the segment boundary, and
- said obtaining the position information comprising establishing, by means of time interpolation, a relative position of the sensor (10) between two segment boundaries, based on a traversing speed of the sensor (10) and the position information.

11. A computer program product comprising instructions for causing a processor system to perform the steps of the method according to claim 10.

## Patentansprüche

1. System zum Zuordnen einer Sensorposition zu einer Bildposition, wobei das System Folgendes umfasst:
- Positionsinformationsmittel (1), die angeordnet sind, um Positionsinformationen einer Sequenz von Sensorpositionen eines Sensors (10) innerhalb einer röhrenförmigen Struktur zu erlangen, wobei die röhrenförmige Struktur einen Dickdarm umfasst, der Sensor (10) ein Endoskop umfasst und eine Sensorposition relativ zu einer strukturellen Eigenschaft der röhrenförmigen Struktur ist; und
- Abgleichmittel (2), die angeordnet sind, um die Sequenz von Sensorpositionen basierend auf den Positionsinformationen und unter Zugrundelegung der strukturellen Eigenschaft der röhrenförmigen Struktur mit einer entsprechenden Sequenz von Bildpositionen abzugleichen, wobei die Bildpositionen Positionen innerhalb einer entsprechenden röhrenförmigen Struktur sind, die durch ein Bild dargestellt wird, und wobei die Bildpositionen relativ zu der strukturellen Eigenschaft der röhrenförmigen Struktur bekannt sind, um Abgleichinformationen zu erlangen;
**dadurch gekennzeichnet, dass**
- die strukturelle Eigenschaft der röhrenförmigen Struktur eine Segmentgrenze zwischen zwei Segmenten des Dickdarms ist, wobei die Positionsinformationen eine Angabe dazu umfassen, wann der Sensor (10) die Segmentgrenze überquert, und
- die Positionsinformationsmittel (1) angeordnet sind, um mittels Zeitinterpolation eine relative Position des Sensors (10) zwischen zwei Segmentgrenzen basierend auf einer Durchquerungsgeschwindigkeit des Sensors (10) und den Positionsinformationen festzustellen.

2. System nach Anspruch 1, weiterhin Zuordnungsmittel (3) umfassend, die angeordnet sind, um ein durch den Sensor (10) an einer bestimmten Sensorposition innerhalb der röhrenförmigen Struktur gemessenes Datenelement (21) basierend auf den Abgleichinformationen einer entsprechenden Bildposition (22) in der entsprechenden röhrenförmigen Struktur zuzuordnen.

3. System nach Anspruch 2, weiterhin Visualisierungsmittel (4) umfassend, die angeordnet sind, um eine Angabe des Datenelements (21) und eine Angabe der entsprechenden Bildposition (22) zu visualisieren.

4. System nach Anspruch 3, wobei die Visualisierungsmittel (4) auf Positionsinformationen reagieren, die sich auf eine aktuelle Position des Sensors (10) beziehen.

5. System nach Anspruch 3, weiterhin Berichterstellungsmittel (5) umfassend, die angeordnet sind, um einen Bericht zu erstellen, der die Angabe des Datenelements (21) und die Angabe der entsprechenden Bildposition (22) enthält.

6. System nach Anspruch 1, wobei die Positionsinformationen (1) räumliche Koordinaten des Sensors (10) umfassen, wobei eine Sequenz von räumlichen Koordinaten, die der Sequenz von Sensorpositionen zugeordnet ist, mindestens einen Teil einer Mittellinie der röhrenförmigen Struktur angibt, wobei die strukturelle Eigenschaft eine Form von mindestens einem Teil der Mittellinie umfasst.

7. System nach Anspruch 6, wobei die Abgleichmittel (2) Ausrichtungsmittel (6) umfassen, die angeordnet sind, um mindestens einen Teil der Mittellinie der röhrenförmigen Struktur mit mindestens einem Teil einer Mittellinie der entsprechenden röhrenförmigen Struktur auszurichten, die durch das Bild dargestellt wird.

8. System nach Anspruch 7, wobei die Ausrichtungsmittel (6) Mittel umfassen, die angeordnet sind, um eine Mittellinie zum Verbessern der Ähnlichkeit zwischen mindestens einem Teil der Mittellinie der röhrenförmigen Struktur und mindestens einem Teil der Mittellinie der entsprechenden röhrenförmigen Struktur, die durch das Bild dargestellt wird, lokal zu dehnen oder zu komprimieren.

9. Bilderfassungsgerät mit dem System nach einem der vorhergehenden Ansprüche.

10. Verfahren zum Zuordnen einer Sensorposition zu einer Bildposition, wobei das Verfahren Folgendes umfasst:
- Erlangen (41) von Positionsinformationen einer Sequenz von Sensorpositionen eines Sensors (10) innerhalb einer röhrenförmigen Struktur, wobei die röhrenförmige Struktur einen Dickdarm umfasst, der Sensor ein Endoskop umfasst und eine Sensorposition relativ zu einer strukturellen Eigenschaft der röhrenförmigen Struktur ist; und
- Abgleichen (42) der Sequenz von Sensorpositionen basierend auf den Positionsinformationen und unter Zugrundelegung der strukturellen Eigenschaft der röhrenförmigen Struktur mit einer entsprechenden Sequenz von Bildpositionen, wobei die Bildpositionen Positionen innerhalb einer entsprechenden röhrenförmigen Struktur sind, die durch ein Bild dargestellt wird, und wobei die Bildpositionen relativ zu der strukturellen Eigenschaft der röhrenförmigen Struktur bekannt sind, um Abgleichinformationen zu erlangen;
**dadurch gekennzeichnet, dass**
- die strukturelle Eigenschaft der röhrenförmigen Struktur eine Segmentgrenze zwischen zwei Segmenten des Dickdarms ist, wobei die Positionsinformationen eine Angabe dazu umfassen, wann der Sensor (10) die Segmentgrenze überquert, und
- das genannte Erlangen der Positionsinformationen das Feststellen einer relativen Position des Sensors (10) zwischen zwei Segmentgrenzen mittels Zeitinterpolation basierend auf einer Durchquerungsgeschwindigkeit des Sensors (10) und den Positionsinformationen umfasst.

11. Computerprogrammprodukt mit Anweisungen, um ein Prozessorsystem zu veranlassen, die Schritte des Verfahrens nach Anspruch 10 auszuführen.

## Revendications

1. Système pour associer une position de capteur à une position d'image, comprenant :
- des moyens d'informations de position (1) agencés pour obtenir des informations de position d'une séquence de positions de capteur d'un capteur (10) à l'intérieur d'une structure tubulaire, la structure tubulaire comprenant un colon, le capteur (10) comprenant un endoscope, une position de capteur étant relative à une caractéristique structurelle de la structure tubulaire ; et
- des moyens de mise en correspondance (2) agencés pour, sur la base des informations de position et en utilisant la caractéristique structurelle de la structure tubulaire, mettre la séquence de positions de capteur en correspondance avec une séquence correspondante de positions d'image, les positions d'image étant des positions à l'intérieur d'une structure tubulaire correspondante représentée par une image et les positions d'image étant connues par rapport à la caractéristique structurelle de la structure tubulaire, pour obtenir des informations de correspondance ;
**caractérisé par** :
- la caractéristique structurelle de la structure tubulaire étant une frontière de segment entre deux segments du colon, les informations de position comprenant une indication précisant quand le capteur (10) traverse la frontière de segment, et
- les moyens d'informations de position (1) étant agencés pour établir, au moyen d'une interpolation dans le temps, une position relative du capteur (10) entre deux frontières de segment, sur la base d'une vitesse de traversée du capteur (10) et des informations de position.

2. Système selon la revendication 1, comprenant en outre des moyens d'association (3) agencés pour associer un élément de données (21) mesuré par le capteur (10) à une position de capteur particulière à l'intérieur de la structure tubulaire à une position d'image correspondante (22) dans la structure tubulaire correspondante, sur la base des informations de correspondance.

3. Système selon la revendication 2, comprenant en outre des moyens de visualisation (4) agencés pour visualiser une indication de l'élément de données (21) et une indication de la position d'image correspondante (22).

4. Système selon la revendication 3, les moyens de visualisation (4) étant réactifs à des informations de position relatives à une position actuelle du capteur (10).

5. Système selon la revendication 3, comprenant en outre des moyens de rapport (5) agencés pour créer un rapport comprenant l'indication de l'élément de données (21) et l'indication de la position d'image correspondante (22).

6. Système selon la revendication 1, les informations de position (1) comprenant des coordonnées spatiales du capteur (10), une séquence de coordonnées spatiales associée à la séquence de positions de capteur étant indicative d'au moins une partie d'une ligne médiane de la structure tubulaire, la caractéristique structurelle comprenant une forme de l'au moins une partie de la ligne médiane.

7. Système selon la revendication 6, les moyens de mise en correspondance (2) comprenant des moyens d'alignement (6) agencés pour aligner au moins une partie de la ligne médiane de la structure tubulaire avec au moins une partie d'une ligne médiane de la structure tubulaire correspondante représentée par l'image.

8. Système selon la revendication 7, les moyens d'alignement (6) comprenant des moyens agencés pour étirer ou compresser localement une ligne médiane afin d'améliorer une similarité entre au moins une partie de la ligne médiane de la structure tubulaire et au moins une partie de la ligne médiane de la structure tubulaire correspondante représentée par l'image.

9. Appareil d'acquisition d'image comprenant le système selon l'une quelconque des revendications précédentes.

10. Procédé d'association d'une position de capteur à une position d'image, comprenant :
- l'obtention (41) d'informations de position d'une séquence de positions de capteur d'un capteur (10) à l'intérieur d'une structure tubulaire, la structure tubulaire comprenant un colon, le capteur comprenant un endoscope, une position de capteur étant relative à une caractéristique structurelle de la structure tubulaire ; et
- sur la base des informations de position et en utilisant la caractéristique structurelle de la structure tubulaire, la mise en correspondance (42) de la séquence de positions de capteur avec une séquence correspondante de positions d'image, les positions d'image étant des positions à l'intérieur d'une structure tubulaire correspondante représentée par une image et les positions d'image étant connues par rapport à la caractéristique structurelle de la structure tubulaire, pour obtenir des informations de correspondance ;
**caractérisé par** :
- la caractéristique structurelle de la structure tubulaire étant une frontière de segment entre deux segments du colon, les informations de position comprenant une indication précisant quand le capteur (10) traverse la frontière de segment, et
- ladite obtention des informations de position comprenant l'établissement, au moyen d'une interpolation dans le temps, d'une position relative du capteur (10) entre deux frontières de segment, sur la base d'une vitesse de traversée du capteur (10) et des informations de position.

11. Produit de programme informatique comprenant des instructions pour amener un système de processeur à effectuer les étapes du procédé selon la revendication 10.
